(19) Europäisches Patentamt / European Patent Office / Office européen des brevets

(11) **EP 3 960 285 B1**

(12) **EUROPEAN PATENT SPECIFICATION**

(45) Date of publication and mention
of the grant of the patent:
**29.04.2026 Bulletin 2026/18**

(21) Application number: **21192963.3**

(22) Date of filing: **25.08.2021**

(51) International Patent Classification (IPC):
***B01F 31/22*** *(2022.01)*

(52) Cooperative Patent Classification (CPC):
**B01F 31/22**

(54) **SELF-BALANCING SHAKER**

SELBSTAUSWUCHTENDE SCHÜTTELVORRICHTUNG

TAMIS VIBRANT À ÉQUILIBRAGE AUTOMATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.08.2020 DK PA202000958**

(43) Date of publication of application:
**02.03.2022 Bulletin 2022/09**

(73) Proprietor: **TI Initiatives ApS
2970 Hørsholm (DK)**

(72) Inventors:
• **Svendsen, Ivan
4400 Kalundborg (DK)**
• **Andreasen, Thomas
2970 Hørsholm (DK)**

(74) Representative: **Budde Schou A/S
Dronningens Tvaergade 30
1302 Copenhagen K (DK)**

(56) References cited:
**WO-A1-2019/222445**

## Description

### FIELD OF THE INVENTION

The present invention relates to a shaker apparatus for agitating, mixing and blending substances, an incubator with such a shaker inside as well as a method for using the shaker apparatus.

### BACKGROUND OF THE INVENTION

Generally, in vitro culture of living cells requires an environment offering acceptable physical and chemical conditions, typically provided by incubators, which keep the temperature and $CO_2$ content in the air constant (often 37°C and 5% $CO_2$, respectively), thereby limiting temperature and acidity fluctuations. In many cases, cells are cultured in liquid culture medium. A widely used and standardised form of container for cells in relatively small volumes of culture medium ($4\mu l$ - 5ml) is known as microtiter plates (MTP). These are available in many different sizes so that one can best meet cell number and experiment design requirements. For some applications, cells require some form of movement which is achieved by shaking the MTPs by, e.g., constant swirling. Typically, this is achieved by placing one or more MTPs on an orbital shaker set to rotate preferably at a 25 mm radius and 225 rpm. These settings may vary depending on the size of the wells, culture medium volume, or even cell number or type. Performing this orbital agitation causes an imbalance in the shaker, since an eccentric movement of the mass is created. This imbalance propagates to the rest of the incubator, if the shaker does not have a balancing means and / or a large mass. Increasing the mass of the shaker is often undesirable.
One solution is to use fixed counterweights situated at specific locations relative to the orbital platform. In a development thereof, the position of the counterweights is adjustable along two axes of the shaker. A further development is to use a servomotor to compensate shakers dynamically by moving the counterweights during or before operation which stabilises the shaker against different loads. Sensors and a control unit are thus required to move the counterweights.
WO 2019/222445A1 describes a counterbalanced shaker apparatus for agitating, mixing or blending substances, the shaker apparatus having a shaker body and the shaker further comprising an eccentric structure having a crank means adapted to provide circular motion, where the eccentric structures each comprises a weight element for counterbalancing the eccentric structure.
The better these shakers become at balancing the eccentric load, the more complicated they have to be, and thus the more expensive the shakers become. They also become more complicated to service and repair. To retain eccentric platform space, the shakers also become larger and heavier.
There is thus a need for a more inexpensive and/or compact orbital shaker.

### SUMMARY OF THE INVENTION

In an aspect of the invention, there is provided a shaker apparatus for agitating, mixing or blending substances, according to claim 1.
Counteraction is thus achieved among the set of eccentric structures of the shaker which set makes up a rotating assembly. The counteraction conceptually achieves net-zero linear forces in an x and y-direction of the plane that is horizontal during use of the shaker.
The crank means can be conceived in a variety of ways. In an embodiment, the circular motion is produced by a crank with a spin axis having a crankpin orbiting the crank spin axis. The crankpin may constructionally be connected by a ball bearing, an intermediary low friction tube such as a brass tube, or a teflon tube or any other friction reducing part. By angular locations is meant the specific point that defines the centre of mass at a specific time along its circular path as measured radially. During a phase, the centre of mass will have occupied each point consecutively and returned to the start. For many embodiments, the angular locations of the centre of mass follow a crankpin orbital movement. However, other solutions are contemplated. For the current disclosure, when crankpin radii is discussed, this should readily be understood to also describe the circular motion radii of the centre of mass of the eccentric structure . When orbital location and orbital velocity are discussed as pertaining to a crankpin, this should also readily be understood to describe more generally the angular location and circular velocity of the centre of mass of the eccentric structure. For illustrative purposes, the outset is taken for example in the crankpin when discussing the figures. However, this is just a preferred embodiment among embodiments of the invention.
In other embodiments, different structures can be conceived. Crank means can be any device that can induce a circular motion to the rack. A structure using pneumatic pistons / actuators or hydraulic pistons / actuators or linear motors can be used to induce two individual reciprocating motions. Preferably, such system has two linear movers that each moves according to linear sinusoidal movement. In other words, they preferably each has a relatively high velocity in the middle of their stroke / movement and a relatively low velocity at the ends of their stroke / movement. Such movement approximates circular movement. Obviously, other combinations of movements be used to design special shaking motion.

It is likewise also possible to use electromagnetic means to provide the circular motion.

By kinematically linked is meant that the rack is connected kinematically to the crank means, such as directly, for the transfer of forces to perform the desired work.

In the specification, the crank means will be discussed as a crank with a crankpin orbiting the crank spin axis. However, as already discussed, the circular motion of the rack can be provided in a variety of ways that do not rely on a crank with a crankpin.

If the crank means and rack are connected directly, the crankpin can rotate relative to the connection of the rack to transfer its orbital motion without its rotation. This is what is called a kinematic pair. Since the crankpin does not transfer its rotation, another degree of freedom is afforded to the rack which is absorbed / restricted / removed by the guide disallowing rack rotation in the plane that is horizontal during use of the shaker.

The counteraction is achieved by predetermining the relative load placements in what may be imagined as a common crank orbit as well as the inertial masses of the eccentric structures. Thus, the shaker is constructed in a manner to ensure that there is dynamic force mitigation among the eccentric structures of a rotating assembly. A rotating assembly is thereby a set of eccentric structures, whose inertial masses are mutually used to counteract each other in a predetermined manner. The rotating assemblies achieve substantially a net zero force in the horizontal plane, when the shaker is in use.

Thereby, as the shaker does not need a traditional counterweight, it can be lightweight. For a shaker with a given rack capacity, using active shaker components as counterweight thus saves weight off of the shaker. A rack has at least one shelf for placing containers with substances. In an embodiment, the rack has at least two shelves for placing containers with substances.

Furthermore, the shaker can be significantly smaller than another shaker with a comparable capacity. Yet further, the shaker may start and stop operation significantly faster because there is less weight to accelerate and decelerate.

Traditionally, a counterweight may be used that is significantly heavier than the eccentric platform which is then located nearer the rotational axis to match the rotational inertia. The invention removes the passive counterweight. The mass of the counterbalancing eccentric masses themselves ensures that unbalanced rotational inertia is significantly avoided.

Depending on the structure of the shaker, torques may arise that need mitigation both above and beyond the counteracted horizontal linear forces. This can be counteracted in a variety of ways. One way is to use a circular rail on top of which the shaker can then oscillate. Another option is to provide the individual eccentric structures vertically above each other and having the centre of their rotation overlapping the referential line.

By eccentric structure is indicated that the mass of the structures moves along an orbital path moved by an eccentrically placed crankpin. Each eccentric structure has a centre of mass that moves in a circle around an axis that is termed a transposed axis in the specification, and the eccentric structures thus all affect the shaker body by their rotation induced by the eccentric placement of the crankpins. The eccentric structures move in circles around their respective paths in matched ways that combine to cancel out the produced forces, and for some embodiments, torques as well.

Shaker apparatus and shaker will be used interchangeably throughout the specification.

The spin and spin velocity are the angular velocity around the centre of rotation. It is identical for two cranks of different sizes that complete a full rotation in the same time. The orbital motion of the crankpins is the circular motion produced, such as around a crank axle during rotation.

By forces is meant linear forces. These oppose and counteract each other during use for all embodiments of the invention by synchronous spin of cranks of eccentric structures of a rotating assembly. For certain preferred embodiments, the torque, which results, when the shaker is in use, around the shaker body referential line of mass also cancels out. This is termed dynamic balance and results, when a second rotating assembly is used.

By crank and crankpin are meant a structure that converts a rotary spin motion of the crank into a rotary orbit motion of the crankpin. Any structure that works for this is useful, such as a traditional crank structure. As is the case for certain embodiments of the invention, several cranks can be part of the same physical structure, such as a crankshaft.

In an embodiment, rotating the crank translates the linked rack along two directions. In an embodiment, moving the crankpin in its orbit moves the linked rack in a circular motion or a linear motion. In an embodiment, the shaker is an orbital shaker.

In an embodiment, the forces generated by the eccentric structures cancel each other out substantially.

In an embodiment, the crank means has a crankpin orbiting a crank spin axis which crankpin connects the crank means to the rack to induce said circular motion.

In an embodiment, the shaker operates at rotational amplitudes having a radius of 0-100mm. In an embodiment, the cranks operate at a spin velocity that produces 0-1500 rpm.

The centres of mass of the eccentric structures have an angular location along their respective circular paths, which angular locations are arranged equiangularly around this common transposed axis, if the transposed axes were rearranged to be colinear. Thereby, the crankpins are arranged equiangularly in their orbits which makes balancing the shaker easier during production and quality control as well as during maintenance. It allows a symmetrical layout that may also use the shaker space better.

In an embodiment, the crankpins of a rotating assembly are arranged in their respective orbits in a manner so that if the cranks were transposed to share a common spin axis with crankpin orientations intact, the crankpins would be arranged equiangularly around this common spin axis. Thereby, the crankpins are arranged equiangularly in their orbits which makes balancing the shaker easier during production and quality control as well as during maintenance.

The centres of mass of each eccentric structure move in a circular motion around a transposed axis when induced by their crankpins. Thereby, an eccentric movement is ensured that allows swirling motion for the substances on the racks.

In an embodiment of the invention, a circle with the referential line at its centre has a periphery intersecting each transposed axis. Thereby, each eccentric structure affects the shaker with torques of equal lengths / arms. In other words, if the forces at work in the eccentric structures are identical, the eccentric structures affect the shaker with equal torque.

In an embodiment of the invention, the eccentric structures have substantially identical inertial masses. In an embodiment, the eccentric structures have substantially identical crankpin orbital radii.

In an embodiment, the referential line substantially intersects with a centre of mass of the shaker body. Thereby, the rotating assembly shares centre of mass with the shaker apparatus, or at least they are on a vertical line. Thereby, if the rotating assembly is balanced dynamically, it does not exchange net torque with the shaker body.

In an embodiment, synchronised spin is induced by a common electro-kinematic chain connected to each crank, the electro-kinematic chain kinematically connecting cranks to coordinated drive means. An electro-kinematic chain denotes: (1) a kinematic chain; or (2) a number of kinematic chains driven by individual motor means that are coordinated electronically by an electronic controller. Either serves to induce a common spin velocity. Drive means is then a motor or a plurality of motors of any useful type such as an electromotor or a diesel motor. Thereby, the spin synchronicity is ensured which ensures shaker durability and effective agitation, mixing or blending. If more than one rotating assembly is used, the electro-kinematic chain coordinates at least both rotating assemblies.

In an embodiment, the electro-kinematic chain comprises at least one of a continuous belt, a chain and an electrical controller. Thereby, an effective structure is used to ensure spin synchronicity.

In an embodiment, a common electro-kinematic chain induces a common spin velocity in the cranks of the rotary assembly.

In an embodiment, a common kinematic chain induces a common spin velocity in the cranks of the rotary assembly. The kinematic chain may be a belt or a chain and it may include rods or other links.

In an embodiment, the cranks are driven by individual axles.

In an embodiment, the cranks are part of a common crankshaft, and the crankpins are connected to the racks through connecting rods. In this embodiment, it is necessary or at least beneficial to remove a degree of freedom introduced by the rod, such as by a guide plate with an orbital channel which may guide the rod end connecting to the rack.

In an embodiment, the shaker comprises a second rotating assembly having a second set of eccentric structures, whose crank means provide circular motion with a second synchronised spin $S_2$, when the shaker apparatus is in use, which second synchronised spin $S_2$ operates at the same spin velocity and the opposite spin direction as the first synchronised spin $S_1$, when the shaker apparatus is in use, whereby torque produced by movement of the eccentric structures oppose and counteract.

The use of a second rotating assembly allows a significant net torque reduction.

Where a second rotating assembly is used, the same features relate to it as to the rotating assembly otherwise described except for the spin direction.

In an embodiment having two rotating assemblies, the centres of mass of all eccentric structures are placed at a mutually equal distance from the referential line.

In an embodiment, the torques generated by the rotating assemblies cancel each other out significantly. In an embodiment, they provide the shaker dynamic balance.

This achieves a mirrored eccentric structure movement which serves to negate torques produced by the individual rotating assemblies completely. In effect, where the set of eccentric structures of a rotating assembly counteract the forces produced among themselves, the first and second rotating assemblies counteract the torques produced among themselves.

By using a second rotating assembly as described, dynamic balance is achieved. Thereby, the shaker experiences no forces or torques by its operation, and spin velocities are only limited by constructional tolerances, i.e. how closely the shaker is constructed to specification. Thereby, a lightweight, long-lasting, compact shaker is achieved.

It has been found that due to eccentric structure masses, the racks can be loaded somewhat unevenly without problem. This also means that the structure itself can be constructed somewhat asymmetrically without falling outside the scope.

By orbital radius is meant the distance between a crank spin axis and the location of the crankpin of that crank. By orbit is meant the path taken by the crankpin around the crank spin axis.

In an embodiment, each eccentric structure centre of mass moves in a circle around a transposed axis, where the transposed axes of the eccentric masses are co-linear. Thereby, the torques produce a steady torque on the shaker. In an embodiment of the invention, racks are divided into digitised structures, whose digits move among each other. Thereby, vertical skewing is further mitigated.

In an embodiment, the referential line denotes a combined centre of mass of the eccentric structures and/or the combined centre of mass of the rotating assemblies.

In an embodiment with two rotating assemblies,

- two transposed axes of the first rotating assembly are arranged on a first line intersecting between them the referential line, and
- two transposed axes of the second rotating assembly are arranged on a second line intersecting between them the referential line, wherein
- the first line is orthogonal to the second line.

Thereby, the eccentric structures move in circles that are arranged essentially 90 degrees relative to each other from the referential line. This balances the forces and torque and is especially advantageous and simple during production, and maintenance is simple and efficient.

In an embodiment having two rotating assemblies, the angular locations of the centres of mass of the eccentric structures are such that during use, they are at a point closest to a shaker referential line simultaneously. Thereby, the torques cancel out completely for this embodiment which ensures vibration-low use of the shaker.

A central concept in the invention is that the cranks have synchronous spin, i.e., identical spin velocity and spin direction. Counter spin indicates identical spin velocity and opposite direction. The synchronous spin of the cranks retains the relative inertial mass locations of eccentric structures through a full rotation of the crank during use of the shaker. This ensures that the rotating assembly retains its predesigned dynamic balance.

A full rotation denotes a full phase. Moving the cranks in phase denotes that the cranks perform a full rotation in the same amount of time.

Orbital velocity denotes crankpin velocity in its orbit, while tangential velocity / speed refers to momentary velocity tangential to the orbit radii.

In an embodiment with three or more eccentric structures, all crank spin axes of the shaker are spaced at equal distances from a referential vertical axis. This orients all loads around a central point relative to each other, and it is thus easier to generate circular motion with same angular torque on each axle. With this arrangement and with the same forces generated by all rotatable axles, forces generated by rotation of the axles counterbalance in the shaker body even better thereby improving shaker balance further.

In a preferred embodiment, the referential vertical axis intersects with the centre of mass of the shaker.

In an aspect of the invention, it relates to an incubator comprising a shaker according to the invention.

Thereby, the low vibration nature of the invention is utilised to gain further advantage.

In an embodiment of the invention in which it relates to an incubator, the racks each has a plurality of shelves, and the incubator further has:

- a plurality of doors matching said plurality of shelves, and
- a rotation mechanism adapted to rotate the shaker inside the incubator.

Thereby, the low-vibration nature of the invention is further utilised to provide a user-friendly eccentric motion incubator rack that only needs to be accessible from one side.

In an embodiment of the invention in which it relates to an incubator, it further has a processor and storage means, where a user can interact with the incubator by

- supplying identification data to the incubator to store a substance on a rack shelf, the processor linking the used rack shelf with said identification data and storing said link in said storage means,
- when the user provides said identification data to the processor, the processor retrieves the stored linked substance position from the storage means, and operates said rotation mechanism to align said rack shelf with a matching door and indicates the door to be opened or opens the relevant door to the user.

Thereby, an especially easy and user-friendly shaker and incubator are provided that leverage the benefits such as the low weight and low vibrations to allow an automated solution that further disinclines errors in the substance-handling process and speeds interaction up and which may even provide compartmentalised access on a single shaker device.

In an aspect of the invention, it relates to using the shaker as described.

In an aspect of the invention, it relates to a method according to claim 13.

In an embodiment, substances are inserted into the racks of at least two of said eccentric structures prior to activating said shaker.

Thereby, the benefits of the invention of a low weight and compact size are utilised.

## BRIEF DESCRIPTION OF THE DRAWINGS

In the following, example embodiments are described according to the invention, where

Figs. 1A-B illustrate a shaker according to an embodiment of the invention,
Figs. 2A-C illustrate operation of a shaker according to an embodiment of the invention, and
Fig. 3A illustrates a shaker according to different embodiments of the invention,
Fig. 3B is a force diagram of the shaker of Fig. 3A,
Fig. 3C is a vector equilibrium diagram of the shaker of Fig. 3A,
Fig. 4 illustrates a shaker according to different embodiments of the invention,
Fig. 5 illustrates a belt drive according to an embodiment of the invention,
Fig. 6 illustrates direct-drive cranks according to an embodiment of the invention,
Fig. 7 illustrates an incubator with a shaker according to an embodiment of the invention,
Fig. 8 illustrates a reciprocating shaker according to an embodiment of the invention,
Fig. 9 illustrates a shaker according to a different embodiment of the invention, and
Fig. 10 illustrates a force diagram of the embodiment of Fig. 4.

## DETAILED DESCRIPTION

In the following, the invention is described in detail through embodiments thereof that should not be thought of as limiting to the scope of the invention.

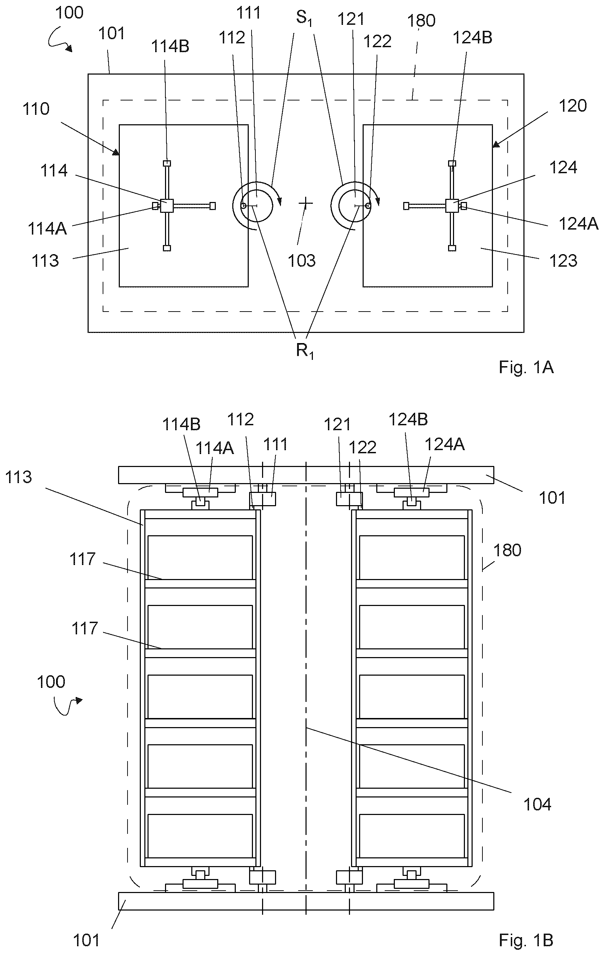

Fig. 1A is a top view of a shaker apparatus 100 according to an embodiment of the invention, and Fig. 1B is a side view of a shaker apparatus 100 of an embodiment of the invention. In the following, the shaker 100 will be described in relation to both and either of these figures.

The shaker 100 has a body 101 that interfaces with the surroundings for example by being placed on a floor. The shaker 100 shown may be symmetrical around a centre of mass 103 of the shaker body 101. A referential line 104 intersects the centre of mass 103 of the shaker body 101 for this situation. However, it is contemplated that depending on placements of drives and kinematic chains and other components as well as the constructional shape of the shaker itself, the centre of mass may be displaced to the side and away from the referential line 104.

The shaker 100 further has a rotating assembly 180 with two eccentric structures 110, 120. The structures are conceptually identical and will be discussed here in relation to the first eccentric structure 110.

The first eccentric structure 110 has a crank 111 with a crankpin 112. When operating, the shaker 100 spins the crank 111 and thus drives an orbital motion of the crankpin 112 at an orbital radius $R_1$. The eccentric structure 110 further has a rack 113 for placing substances for agitating, mixing and blending. Preferably, t , as shown, the rack has several individual shelves 117 for substance placement. The rack 113 is connected to the crankpin 112 and follows it in the plane. The rack 113 is attached to a guide 114 that is attached to the shaker body 101. The guide 114 passively allows translation along the two horizontal axes while supporting the weight of the rack and preventing horizontal rotation. The shown embodiment has a guide 114 in the shape of a dual rail. Thus, the rail comprises body connectors 114A attached firmly to the shaker body 101 and rack connectors 114B attached firmly to the rack, while the two rails are angled perpendicular to each other and each allows translation along one direction each.

When the crank 111 is driven by a motor (not shown), the first eccentric structure 110 thus moves with the orbital movement of the crankpin 112. The orbital movement is a circular motion that can be understood as a two-dimensional movement i.e. translation in the plane. The planar translation is performed in the horizontal plane, when the shaker apparatus 100 is in use.

The circular motion of the first eccentric structure 110 produces the desired shaking or swirling movement of the rack 113 that agitates, mixes and/or blends any substances placed in the rack 113. This circular motion also produces an equal reactionary force, but which, if unmitigated, travels to the shaker body 101 and may vibrate, shake or rock the shaker 100.

The reactionary forces are counteracted and thus mitigated by the structure as shown. The dynamic counteraction is achieved for the rotating assembly 180 by including at least one other eccentric structure, here the second eccentric structure 120, which counteracts the forces exerted by the first eccentric structure 110 on the shaker body 101.

The second eccentric structure 120 counteracts the first eccentric structure 110 by ensuring that the cranks 111, 121 of each structure 110, 120 follow a synchronous spin $S_1$ which means that they have identical spin velocities in the same direction around parallel axes. Thus, they stay aligned during use of the shaker.

In the embodiment shown in Figs. 1A and 1B, the counteraction is further ensured by the orbital radii of the crankpins being identical ($R_1$) and the crankpins being located opposite each other in their respective orbits.

The forces mitigated by the shown structure are what is typically termed the x-component and y-component. A torque may still remain, whereby the rotating assembly seeks to rotate the shaker body 101 and can be mitigated in other ways, such as installing the shaker on a circular rail or building the shaker sufficiently robust. Achieving counteraction according to the invention can be performed in a variety of ways that will be explored further in relation to other figures.

Thereby, a compact shaker 100 is produced that has a low weight in relation to the amount of substance that can be placed in the racks of the shaker 100. This then results in a lightweight and spacious shaker.

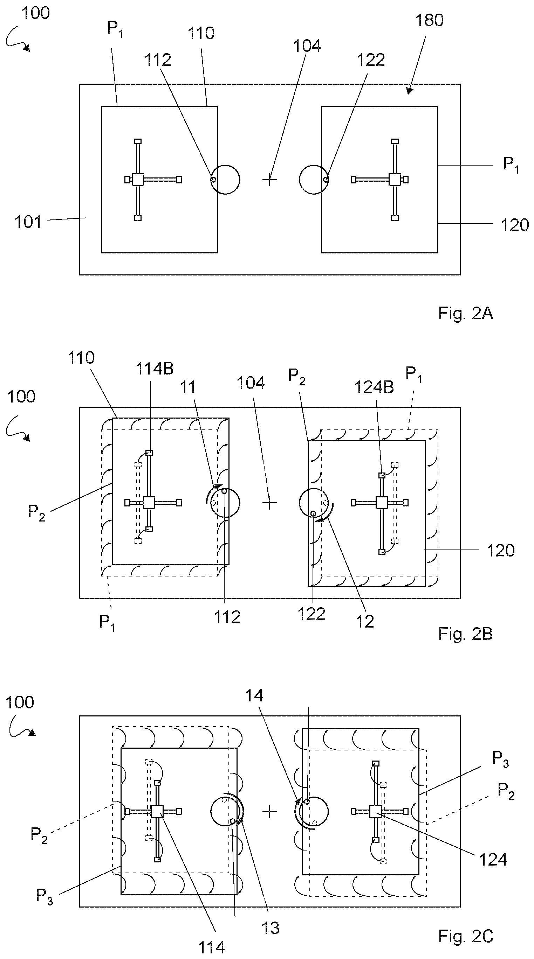

Figs. 2A-2C show the movement of the eccentric structures of a rotating assembly of an embodiment of the invention, such as the one shown in Fig. 1A.

Fig. 2A shows the eccentric structures 110, 120 of a rotating assembly 180 in a rest position $P_1$ corresponding to the position shown in Fig. 1A. The crankpins 112, 122 are located at the point of their orbits farthest from the referential line 104 of the shaker body 101.

In Fig. 2B, the shaker is in use and the rotating assembly 180 in position $P_2$. The cranks are spun a quarter rotation which moves the crankpins a quarter orbit relative to the rest position $P_1$, as indicated by first orbit path 11 and second orbit path 12. The dual rail guides 114, 124 translate the eccentric structures 110, 120 along both individual rails which is seen by the new position of the rack connectors 114B, 124B. As shown, they ensure that the eccentric structure does not rotate, thus constraining motion to ensure a matched movement between the two eccentric structures 110, 120.

A combined path of the first orbit path 11 and second orbit path 12 cancels out with each other. Since the eccentric structures have similar masses, the forces cancel out as well.

This is furthermore the case at every point in time. To see this more clearly, one may imagine first breaking the first orbit path 11 and second orbit path 12 down into infinitesimally small straight vectors. Secondly, one may overlay each straight vector from the first orbit path 11 with its time-matched straight vector from the second orbit path 12, and one will see that they are exactly opposite in direction and length.

The rotating assembly then always produce combined insubstantial x and y directional forces to the shaker body, though the individual eccentric structures 110, 120 are unbalanced.

Fig. 2C shows the rotating assembly with its eccentric structures in a third position $P_3$, in which the eccentric structures have moved an arbitrary orbit length since $P_2$, $P_2$ being shown with dashed lines. Again, the dual rails 114, 124 ensure a constrained planar translation.

Because of the arbitrary orbit paths 13, 14, it is perhaps even more apparent that it is not a coincidence that the forces match.

For the forces to cancel out as described for rotating assemblies with identical crankpin orbit radii, the mass of the racks should be at least quite similar, preferably at least substantially identical. In use, it is advantageous to load the racks as equally as possible, although it has been found that the weight of the eccentric structures 110, 120 provides substantial baseline mass so that equal loading is not strictly necessary.

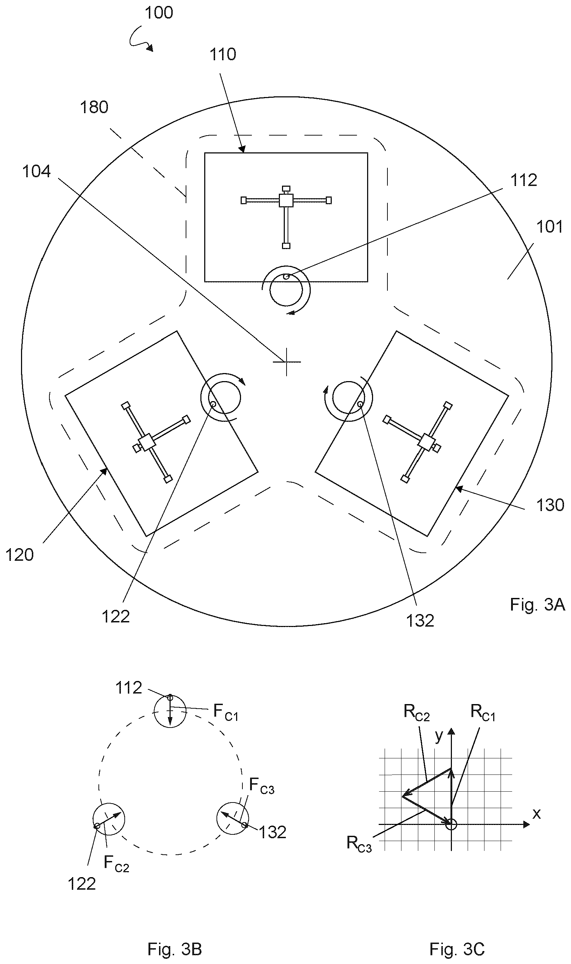

Fig. 3A illustrates a shaker 100 according to different embodiment of the invention having a body 101. The shaker 100 has three eccentric structures 110, 120, 130, each being conceptually identical to the ones hitherto described, that is, they have a crank, a crankpin 112, 122, 132, a rack and guide and substantially identical masses before adding of any substances to the racks. The eccentric structures are arranged radially symmetrically around the referential line 104.

In the shown embodiment, the crankpins 112, 122, 132 are not located oppositely in their orbits in a paired manner, as was shown with Figs. 1-2. Instead, the crankpins are placed equiangularly around the orbit.

Thereby, the rotating assembly of Fig. 3 achieves counteraction among three eccentric structures 110, 120, 130. The vector calculations for the forces are more complicated than for embodiments of Figs. 1-2, but still at least substantially cancels out for the rotating assembly 180. This will be shown in relation to Figs. 3B-C.

Fig. 3B is a force diagram of the shaker of Fig. 3A. The force diagram illustrates the direction and amplitude of the forces at play among the different eccentric structures 110, 120, 130.

The crankpins 112, 122, 132 move in orbits and are thus not individually at rest. Instead, the force $F_{C1}$ is the centripetal force in the crankpin pulling the crankpin towards the centre of rotation without which the momentary tangential velocity of the crankpin would move it in straight line (not shown).

Fig. 3C is a vector equilibrium diagram showing how the centripetal forces affect the shaker body 101 of Fig. 3A. Note that they are opposite to the ones shown in Fig. 3B. Although the forces transplant from the cranks through to the shaker body 101, they meet in the shaker body 101 and cancel out substantially before the shaker body can be affected relative the environment. More generally, the forces of the rotating assembly 180 can be expressed as:

$$\sum_n F_{Cn} = 0$$

The number of eccentric structures is denoted by n for a given rotating assembly 180. In Figs. 3A-C, there are three eccentric structures 110, 120, 130 moving according to a synchronised crank spin.

By using a shaker like the one shown in Fig. 3, a compact design can be achieved for which the forces cancel out.

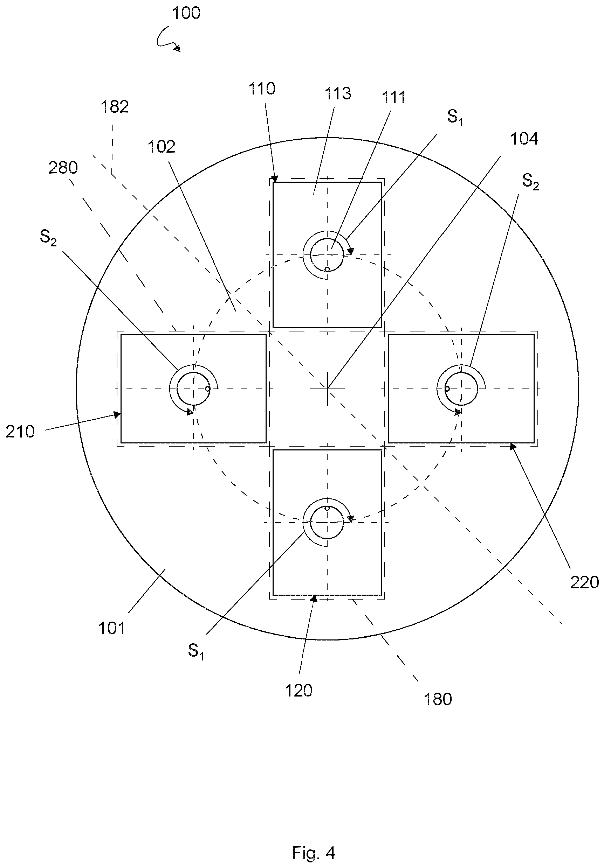

Fig. 4 illustrates a shaker 100 according to different embodiment of the invention having a body 101. The shaker of Fig. 4 has four eccentric structures 110, 120, 210, 220 each with a crank and crankpin, a rack and a guide. The shaker body 101 is constructed radially symmetrically around its referential line 104.

The general layout of the eccentric structures will be briefly discussed using the example of the first eccentric structure 110. The crank 111 is provided directly beneath the rack 113, with the crankpin 112 connecting to the rack centre of mass. The guide (not shown) can be mounted at any place, and preferably, if using a dual rail structure, by providing two rails along a first direction and two others along a second direction perpendicular to the first direction to form a square around the guide. This structure may be especially constructionally sound and wear-resistant, since the rails can be close to the perimeter of the rack. Further, it may also be a safe and compact structure, since the cranks are hidden beneath (and/or above) each rack.

The embodiment shown in Fig. 4 has a rotating assembly 180 hereinafter called the first rotating assembly 180 comprising two eccentric structures 110, 120 and a second rotating assembly 280 comprising two eccentric structures 210, 220. Each rotating assembly 180, 280 is individually counteracting as hitherto described. The eccentric structures have substantially identical unloaded masses.

A circle 102 has the shaker body 101 referential line 104 as its centre. The circle 102 intersects all four crank spin axes.

As can be seen, the crankpins 112, 122 of the first rotating assembly 180 are both closest to the referential line 104 among points along their orbit. They move according to a synchronised spin $S_1$.

The crankpins 212, 222 of the second rotating assembly 280 are also at the point closest to the referential line 104 among points along their orbit. During operation, they move according to a second synchronised spin $S_2$.

The combination of the first rotating assembly 180 and the second rotating assembly 280 cancels out any torques produced by the individual rotating assemblies 180, 280 and thus achieves dynamic balance for the shaker. When the forces acting on the shaker are not balanced relative to the centre of mass of the eccentric structures that may coincide with the referential line 104, a torque acts on the shaker body. As described for Fig. 1, this can be compensated for by placing the shaker body on a circular bearing rail, where the shaker will then oscillate. However, in the embodiment shown in Fig. 4, this is not necessary, since not only the forces but the torques of the shaker cancel out. Thus, the shaker is dynamically counterbalanced.

That the dynamic balance is achieved by counterbalancing torques among rotating assemblies may be understood by referring to the mirror line 182. The orbital path of the first eccentric structure 110 matches and mirrors the orbital path of a third eccentric structure 210 exactly, and the same can be said for the second 120 and fourth 220 eccentric structures.

In the shown embodiment, the two rotating assemblies 180, 280 rotate in opposite directions which is preferable. For some embodiments, it may not be necessary. In other words, $S_1$ and $S_2$ can be identical.

Dynamic balancing of the shaker can be achieved by many structural arrangements that falls within the scope of the invention. The embodiment shown in Fig. 4 is the special case, being a preferred embodiment, where crankpin orbit radii are identical, crankpins are arranged to all be nearest the referential line at the same time, eccentric structure centres of mass are arranged equiangularly around the shaker referential line and at the same distance therefrom, and there are $2^n$ eccentric structures arranged in pairs.

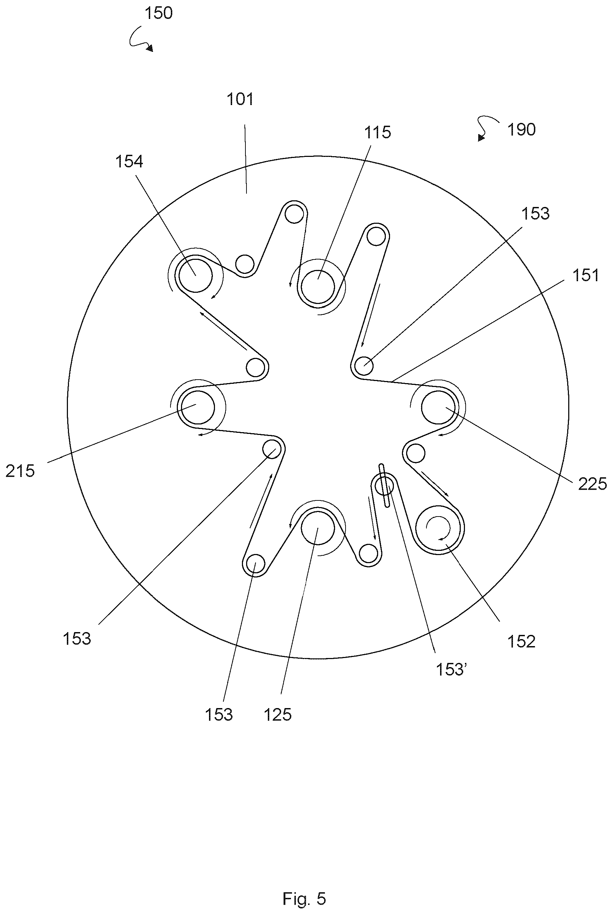

Fig. 5 is a top view of a belt drive 150 on a shaker according to an embodiment of the invention.

The rotating assembly have the axle spin velocities of its cranks synchronised by an electro-kinematic chain 190 in the shape of a belt drive 150. An electro-kinematic chain 190 denotes a kinematic chain which is constrainably controlled by kinematic linkages as well as optionally electrical signals.

As such, the spin velocity $S_1$ may be synchronised between two individual kinematic chains or links driven individually and controlled commonly by an electrical controller to ensure synchronous crank spin. the spin can also be synchronised using a traditional kinematic chain, whose crank spin velocities are kinematically linked to a single drive. Thus, by using an electro-kinematic chain, coordination of spin velocities is ensured.

The belt- drive 150 is placed above the rotating assemblies (not shown). Placing it above may stabilise the shaker body 101 through the weight of the belt drive 150 mechanism. Alternatively, the belt drive may be placed below the rotating assemblies which may reduce vibrations in the belt drive itself as well as reduce weight.

The belt drive has a belt 151 driven by a motor axle 152 and a number of roller pins 153. A number of roller pins 153 can be adjustable roller pins 153' adjustable to tighten the belt 151 against imprecise belt length tolerances and/or over time if the belt expands.

The belt drives four crank axles 115, 125, 215, 225 which in turn drive the aforementioned cranks, crankpins and thus eccentric structures (not shown). The axles are all of matched diameters which serve to coordinate their spin velocities. Furthermore, opposing crank axles 115, 125 and 215, 225, respectively, rotate with synchronous spin.

A fifth axle 154 is also provided which extends past the rotating assemblies to a second belt drive beneath the rotating assemblies (not shown).

The second belt drive (not shown) may essentially mirror the belt drive 150 of Fig. 5, where the fifth axle 154 takes the role of motor axle (and where there is no need for a sixth axle). The fifth axle 154 then drives the second belt drive and second axles being co-axial with the crank axles 115, 125, 215, 225 shown in Fig. 5. This second arrangement of crank axles may be opposite ends of the crank axles driven as described above, or it may be other axles attached to the eccentric structures in another manner. By using a fifth axle 154 to drive a second belt drive, the eccentric structures are provided with a driving force from a top and a bottom which stabilises against twisting, skewing and vibration of the eccentric structures and shaker itself. A belt is a useful choice because the diameters of the crank axles are not susceptible to wear which ensures that the spin velocities stay synchronised.

A different type of kinematic chain can be provided, such as an actual chain, cogs, levers or any other convenient type of kinematic chain. It is preferable to use a type, where wear on the kinematic chain does not de-synchronise the spins of the axles 115, 125, 215, 225.

A chain may be useful because the axles are kept fixed by teeth ensuring the axles are not accidentally brought out of alignment.

A toothed belt may be especially advantageous for these reasons.

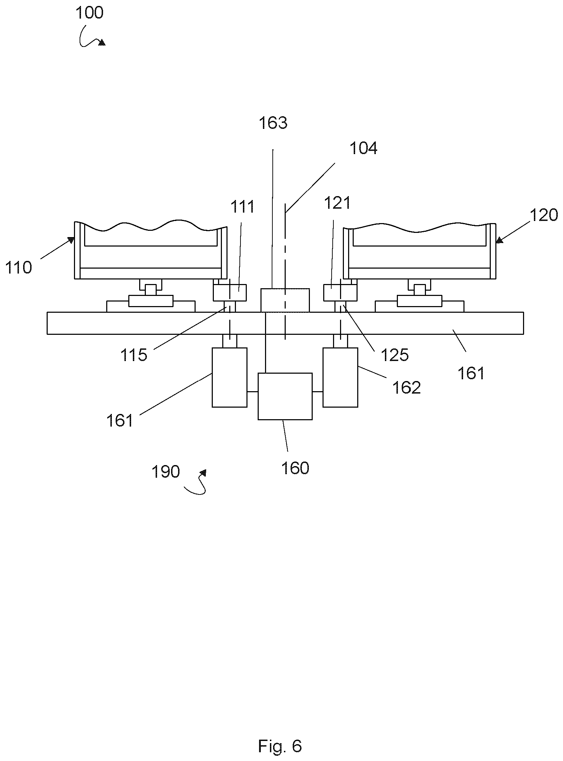

Fig. 6 illustrates a shaker 100 with an electro-kinematic chain 190 using direct-drive cranks 111, 121 according to an embodiment of the invention. In the shown embodiment, a first motor 161 drives a first axle 115, while a second motor 162 drives a second axle 125. Then, these move the eccentric structures 110, 120 as otherwise described. The motors 161, 162 are controlled by a common electrical controller 160.

This may be an easy solution that can be repaired by transmitting an electrical signal to correct position of crankpins dynamically which may avoid the need for technician repair or parts replacement. It may also reduce motor size requirements and may thus result in a less bulky system by for example placing the motors beneath the racks between or next to guides.

In an embodiment, the controller 160 is connected to sensing means 163 which asses the synchronicity of the rotating assembly periodically or continually. If somehow the eccentric structures of a rotating assembly fall slightly out of sync, the sensor can detect either the mismatch between the expected location of an eccentric structure at a given time (measuring position of a crankpin or a rack, for example) or the resultant forces, such as vibration. The sensing means 163 can then be a vibration sensor or an infrared sensor. When a misalignment is detected, it can be readjusted by the signals transmitted by the electrical controller 160.

This provides a relatively easy way of ensuring optimal force cancellation by continually seeking misalignment reduction as well as potentially error-reducing alignment during production.

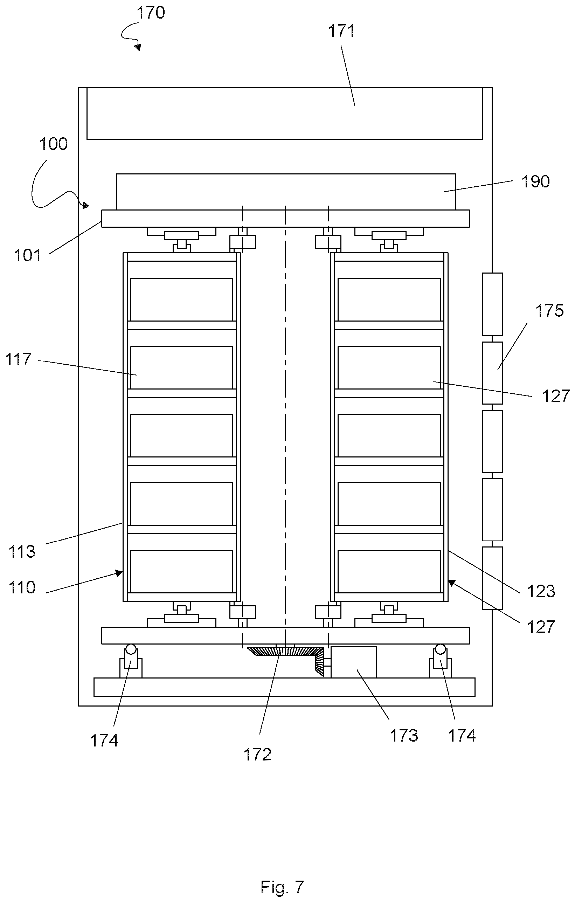

Fig. 7 illustrates shaker 100 inside an incubator 170. Inside the incubator 170, the temperature and preferably atmosphere are controlled by an atmosphere controlling means 171. Gas contents and perhaps pressure can be controlled.

An electro-kinematic chain 190 drives the cranks 111, 121 to move eccentric structures 110, 120 with synchronous spin.

The shaker body 101 can rotate relative to the incubator by way of a rotary drive 172 driven by a motor 173. A circular guide 174 allows free rotation of the shaker 100.

By providing a shaker 100, which can rotate in an automated manner, the shaker 100 can be placed in an incubator 170 with doors 175 in a single side while still having several individual racks 113, 123.

At least one door 175 is provided in the incubator wall to access substances.

Preferably a door 175 is provided for each rack shelf 117. This allows users to open to only the specific samples or substances to which they need access, at a given time. This ensures minimal disturbance of the rest of the controlled environment of the incubator. Further, it allows a user to identify themselves to the incubator using a code or card or other identifying information after which only relevant access is granted. Thereby, fewer errors can occur due to inexperienced users sharing incubator with advanced users. Depending on the substances, this may further provide extra safety.

Preferably, the shaker 100 has a second rotating assembly (not shown) as described for Fig. 4 which ensures rotational inertia stability. This allows the shaker 100 to rotate relatively freely while operating which means it can keep rotating or stop and allow residual eccentric structure rotation, while the shaker is rotated to the desired orientation to allow a user to retrieve substances or insert substances into the shaker 100. This increases shaker working time.

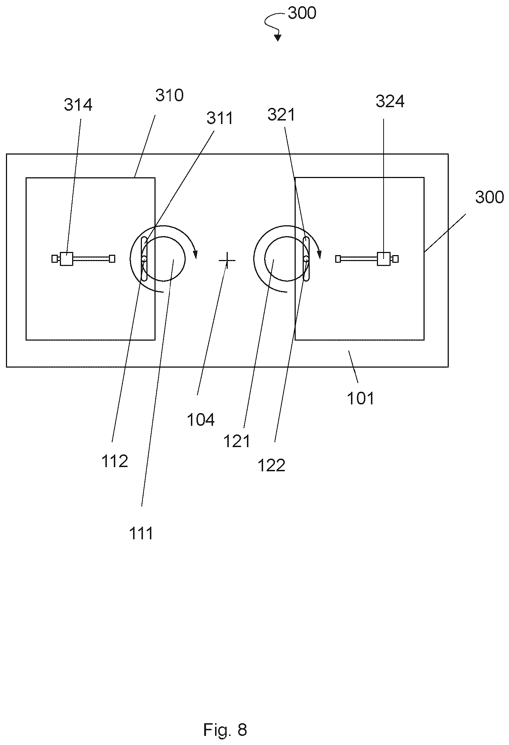

Fig. 8 illustrates a reciprocating shaker 300 according to an embodiment of the invention. The reciprocating shaker 300 is structurally very similar to the hitherto described shakers. It has reciprocating structures 310, 320 that move along one direction instead of the hitherto described two directions. This allows agitating, mixing or blending substances in a different manner that may be advantageous in some situations. It is achieved by connecting the reciprocating structures through conversion slots 311, 312 to crankpins 112, 122 connected to cranks 111, 121.

The conversion slots 311, 312 transmit motion from the crankpins 112, 122 to the rest of the reciprocating structures 310, 320 in the direction that intersects the referential line 104 denoting the combined centre of mass of the reciprocating structures. However, it also absorbs or negates crankpin 112, 122 movement along a direction perpendicular to that direction. The shown shaker 300 thus only moves its reciprocating structures in a colinear direction and in a mirrored fashion which means the conversion guides are always at substantially identical distance to the referential line 104. Any number of reciprocating structures can be used and will be in dynamic balance, when they move in such a mirrored fashion.

The linear guides 314, 324 block horizontal rotation and horizontal sideways translation thus only allowing movement in the colinear direction.

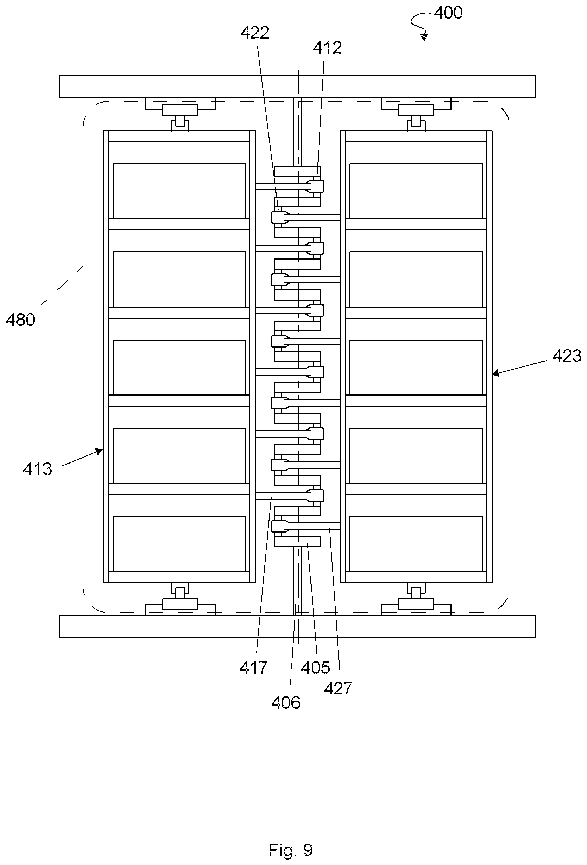

Fig. 9 illustrates a shaker 400 according to a different embodiment of the invention. Generally, the shaker 400 uses a single drive axle to move all racks of a rotating assembly 480.

Cranks 411, 121 are part of an integrated crankshaft 405 that have the crankpins 412, 422 on a common axle 406. Then, connecting rods 417, 427 connect racks 413, 423 to their crankpins 412, 422 of the crankshaft 406 to transmit motion. This allows a compact design as well as assurance that the crankpins are never desynchronised.

The shown embodiment has rod guides (not shown) that allow transmitting orbital crankpin velocity to a similar rotary motion at the racks to ensure a rack rotation as hitherto described. Said rod guides can take the shape of orbital guides which may be circular or oval or other shapes that are efficient to transfer the involved forces.

The eccentric structures 410, 420 each has at least one such connecting rod 417, 427 and may have several as shown. By having several, vertical stability is increased.

Another embodiment is contemplated, where the eccentric structures overlap in the vertical direction which may provide an especially compact design, because all racks of a shaker can thus be accessed from one side and further shaker rotating means becomes unnecessary. Such an embodiment is especially useful when using a crankshaft 406 like shown in Fig. 9.

A piston-like embodiment is considered as well, where racks reciprocate like described with Fig. 8 using a simple link between the connecting rod and rack, and where such a reciprocating structure uses a linear guide (not shown).

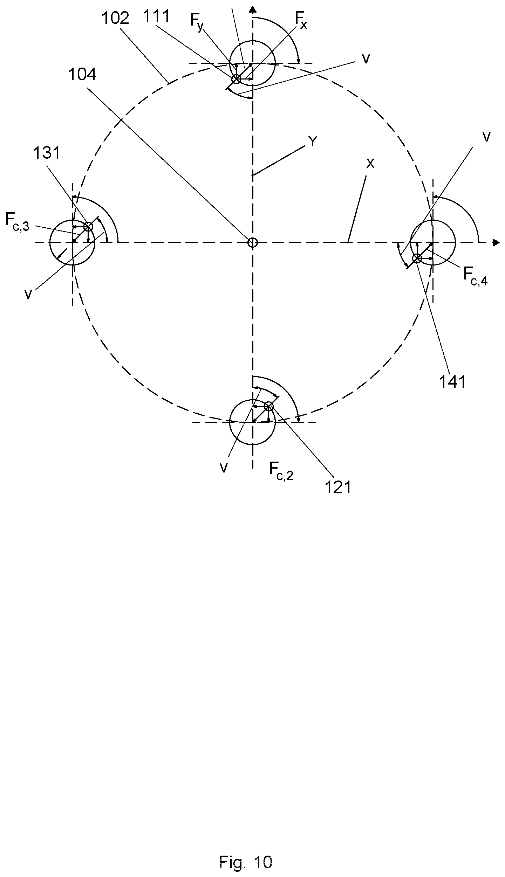

Fig. 10 shows a breakdown of the forces at work in the preferred embodiment otherwise shown in Fig. 4. The crankpins are accelerated towards the crank spin axes by a centripetal force. The centripetal force acting on the crankpin 111 of the first eccentric structure (not shown) is shown to be broken down into an x-component and a y-component. The same is done for all four centripetal forces.

The situation depicted is one in which a time t has passed, since all crankpins were in their innermost positions. Given their relative directional rotation and synchronised spin velocity, they have all moved an angle v.

Calculating the forces, as seen on the figure, towards the left and upwards being positive and towards the right and downwards being negative, the following results (for brevity, '1', '2', '3' and '4' denotes the first 111, second 121, third 131 and fourth 141 crankpins, respectively, in the equations):

The forces acting in the X-direction can be calculated as:

$$F_x' = F_{x,c1}' + F_{x,c2}' + F_{x,c3}' + F_{x,c4}'$$

Which results in:

$$F_x' = -F_C \cos v + F_C \cos v + F_C \sin v - F_C \sin v$$

And thereby:

$$\underline{F_x' = 0}$$

The forces acting in the Y-direction can be calculated as:

$$F_y' = F_{y,c1}' + F_{y,c2}' + F_{y,c3}' + F_{y,c4}'$$

Which results in:

$$F_y' = -F_C \sin v + F_C \sin v - F_C \cos v + F_C \cos v$$

And thereby:

$$\underline{F_y' = 0}$$

It can be noted that the above X-components and Y-components cancel out among the eccentric structures of each rotating assembly and so works out for all embodiments of the invention.

The torque acting on the shaker as created by the eccentric structures can be calculated as follows. Firstly, the torque produced around the referential line 104 is identical for crankpins 111 and 121, which is ('O' marks the centre of mass in the equations):

$$\tau'_{,1} = \tau'_{,2} = -F_C \cos v \left( x \atop \sin v \right) - F_C \sin v \left( d - y \atop \cos v \right)$$

Which is reduced:

$$\tau'_{,1} = \tau'_{,2} = -F_C \sin v \cos v - F_C d \sin v + F_C \sin v \cos v$$

And again:

$$\tau'_{,1} = \tau'_{,2} = -F_C d \sin v$$

Secondly, the torque around the referential line 104 is identical for crankpins 131 and 141, which is:

$$\tau'_{,3} = \tau'_{,4} = F_C \sin v \left( d - x \atop \cos v \right) + F_C \cos v \left( y \atop \sin v \right)$$

Which is reduced

$$\tau'_{,3} = \tau'_{,4} = F_C d \sin v - F_C \sin v \cos v + F_C \sin v \cos v$$

And again:

$$\tau'_{,3} = \tau'_{,4} = F_C d \sin v$$

Combining all gives

$$\tau' = - F_C d \sin v - F_C d \sin v + F_C d \sin v + F_C d \sin v$$

And thereby:

$$\underline{\underline{\tau' = 0}}$$

Thereby, it is shown that a dynamically balanced shaker apparatus is achieved by a preferred embodiment of the invention.

## Claims

1. A shaker apparatus (100) for agitating, mixing or blending substances, the shaker apparatus (100) having a shaker body (101) and the shaker (100) further comprising:

   - an eccentric structure (110) having:

      - a crank means (111) adapted to provide circular motion,
      - a rack (113) for holding trays or containers and supported by the shaker body (101) through a guide (114) that disallows rack rotation in the plane of the circular motion of the crank means (111), the rack being kinematically linked to said crank means (111) to receive said circular motion, where a centre of mass of the eccentric structure (110) moves in said circular motion around a transposed axis, when the crank means moves the rack in the circular motion,

- wherein said shaker apparatus (100) comprises a set of such eccentric structures (110, 120) forming a first rotating assembly (180) whose crank means (111, 121) provide circular motion having a first synchronised spin ($S_1$), when the shaker apparatus (100) is in use, where said rotating assembly (180) has a centre of mass at least substantially on a referential line (104) normal to the plane of the circular motion, and **characterized in that** the centres of mass of the eccentric structures (110, 120) have an angular location along their respective circular paths, which angular locations are arranged equiangularly around a common transposed axis, **in that** the transposed axes are rearranged to be colinear,

wherein relative angular locations of the centres of mass of the eccentric structures (110, 120) along the path of their circular motion are predetermined for the rotating assembly (180) so that when the shaker apparatus (100) is in use, forces produced by movement of the eccentric structures oppose and counteract.

2. A shaker apparatus (100) according to claim 1, wherein the crank means (111) has a crankpin (112) orbiting a crank spin axis which crankpin (112) connects the crank means to the rack to induce said circular motion.

3. A shaker apparatus (100) according to any of claims 1-2, where a circle with the referential line (104) at its centre has a periphery (102) intersecting each transposed axis.

4. A shaker apparatus (100) according to any of claims 1-3, where said crank means (111) is a crank (111) with a crankpin (112) and a spin axis parallel to the referential line (104), the crank converting its spin velocity to orbital velocity of the crankpin (112).

5. A shaker apparatus (100) according to any of claims 1-4, wherein synchronised spin is induced by a common electro-kinematic chain (190) connected to each crank, the electro-kinematic chain kinematically connecting cranks to coordinated drive means.

6. A shaker apparatus (100) according to any of claims 1-5, wherein the electro-kinematic chain (190) comprises at least one of a continuous belt (150), a chain and an electrical controller (160).

7. A shaker apparatus (100) according to any of claims 1-6, comprising a second rotating assembly (280) having a second set of eccentric structures (210, 220), whose crank means provide circular motion with a second synchronised spin ($S_2$), when the shaker apparatus (100) is in use, which second synchronised spin ($S_2$) operates at the same spin velocity and the opposite spin direction as the first synchronised spin ($S_1$), when the shaker apparatus (100) is in use, whereby torques produced by movement of the eccentric structures (110, 120, 210, 220) oppose and counteract.

8. A shaker apparatus according to claim 7, where:

   - two transposed axes of the first rotating assembly are arranged on a first line intersecting between them the referential line (104), and
   - two transposed axes of the second rotating assembly are arranged on a second line intersecting between them the referential line (104), wherein
   - the first line is orthogonal to the second line.

9. A shaker apparatus (100) according to any of claims 7-8, wherein the angular locations of the centres of mass of the eccentric structures (110, 120) are such that during use, they are each at a point closest to a referential line simultaneously.

10. An incubator comprising a shaker (100) according to any of claims 1-9.

11. An incubator according to claim 10, where the racks each has a plurality of shelves, and where the incubator further has:

   - a plurality of doors matching said plurality of shelves, and
   - a rotation mechanism adapted to rotate the shaker (100) inside the incubator.

12. An incubator according to any of claims 10-11 further having a processor and storage means, where a user can interact with the incubator by:

   - supplying identification data to the incubator to retrieve a rack for storage of a substance on the rack shelf, the processor linking the used rack shelf with said identification data and storing said link in said storage means,
- providing said identification data to the processor, whereby the processor retrieves the stored linked substance position from the storage means and operates said rotation mechanism to align said rack shelf with a matching door and indicates the door to be opened or opens the relevant door to the user.

13. A method comprising:

- providing a shaker apparatus (100) for agitating, mixing or blending substances, according to any of claims 1-9,
- activating the shaker by moving the crank means, thereby moving the rack in the circular motion, the centre of mass of the eccentric structure (110) thus moving in said circular motion around a transposed axis,
- wherein activating said shaker apparatus (100) moves a set of such eccentric structures (110, 120) thus forming a first rotating assembly (180), whose crank means (111, 121) provide circular motion having a first synchronised spin ($S_1$), where said rotating assembly has a centre of mass at least substantially on a referential line (104) normal to the plane of the circular motion, and where relative angular locations of the centres of mass of the eccentric structures (110, 120) are predetermined for the rotating assembly (180) so that forces produced by movement of the eccentric structures oppose and counteract.

14. A method according to claim 13 comprising the step of inserting substances into the racks of at least two of said eccentric structures.

**Patentansprüche**

1. Schüttelvorrichtung (100) zum Rühren, Mischen oder Vermengen von Substanzen, wobei die Schüttelvorrichtung (100) einen Schüttelkörper (101) aufweist und die Schüttelvorrichtung (100) ferner Folgendes umfasst:

- eine exzentrische Struktur (110), die Folgendes aufweist:

- eine Kurbeleinrichtung (111), die dazu ausgelegt ist, eine kreisförmige Bewegung bereitzustellen,
- ein Rack (113) zur Aufnahme von Schalen oder Behältern, das von dem Schüttelkörper (101) durch eine Führung (114) getragen wird, die eine Drehung des Racks in der Ebene der kreisförmigen Bewegung der Kurbeleinrichtung (111) verhindert, wobei das Rack kinematisch mit der Kurbeleinrichtung (111) verbunden ist, um die kreisförmige Bewegung aufzunehmen, wobei ein Massenschwerpunkt der exzentrischen Struktur (110) sich in der genannten Kreisbewegung um eine versetzte Achse bewegt, wenn die Kurbeleinrichtung das Rack in der Kreisbewegung bewegt,

- wobei die Schüttelvorrichtung (100) einen Satz solcher exzentrischen Strukturen (110, 120) umfasst, die eine erste rotierende Anordnung (180) bilden, deren Kurbeleinrichtung (111, 121) eine kreisförmige Bewegung mit einer ersten synchronisierten Drehung ($S_1$) erzeugen, wenn die Schüttelvorrichtung (100) im Gebrauch ist, wobei die rotierende Anordnung (180) einen Massenschwerpunkt hat, der zumindest im Wesentlichen auf einer Bezugslinie (104) senkrecht zur Ebene der kreisförmigen Bewegung liegt, und **dadurch gekennzeichnet, dass** die Massenschwerpunkte der exzentrischen Strukturen (110, 120) eine Winkelposition entlang ihrer jeweiligen kreisförmigen Bahnen haben, wobei die Winkelpositionen gleichwinklig um eine gemeinsame versetzte Achse angeordnet sind, indem die versetzten Achsen umgeordnet sind, so dass sie kolinear sind, wobei die relativen Winkelpositionen der Massenschwerpunkte der exzentrischen Strukturen (110, 120) entlang des Pfades ihrer kreisförmigen Bewegung für die rotierende Anordnung (180) vorbestimmt sind, so dass, wenn die Schüttelvorrichtung (100) in Gebrauch ist, die durch die Bewegung der exzentrischen Strukturen erzeugten Kräfte sich entgegengesetzt verhalten und entgegenwirken.

2. Schüttelvorrichtung (100) nach Anspruch 1, wobei die Kurbeleinrichtung (111) einen Kurbelzapfen (112) aufweist, der die eine Kurbeldrehachse umkreist, wobei der Kurbelzapfen (112) die Kurbel mit dem Rack verbindet, um die Kreisbewegung zu bewirken.

3. Schüttelvorrichtung (100) nach einem der Ansprüche 1-2, wobei ein Kreis mit der Bezugslinie (104) in seinem Zentrum einen Umfang (102) hat, der jede versetzte Achse schneidet.

4. Schüttelvorrichtung (100) nach einem der Ansprüche 1-3, wobei die Kurbeleinrichtung (111) eine Kurbel (111) mit einem Kurbelzapfen (112) und einer Drehachse parallel zur Bezugslinie (104) ist, wobei die Kurbel ihre Drehgeschwindigkeit in eine Umlaufgeschwindigkeit des Kurbelzapfens (112) umwandelt.

5. Schüttelvorrichtung (100) nach einem der Ansprüche 1-4, bei der die synchronisierte Drehung durch eine gemeinsame elektrokinematische Kette (190) induziert wird, die mit jeder Kurbel verbunden ist, wobei die elektrokinematische Kette die Kurbeln kinematisch mit koordinierten Antriebsmitteln verbindet.

6. Schüttelvorrichtung (100) nach einem der Ansprüche 1-5, wobei die elektro-kinematische Kette (190) mindestens ein Endlosband (150), eine Kette und eine elektrische Steuerung (160) umfasst.

7. Schüttelvorrichtung (100) nach einem der Ansprüche 1-6, umfassend eine zweite rotierende Baugruppe (280) mit einem zweiten Satz exzentrischer Strukturen (210, 220), deren Kurbeleinrichtungen eine kreisförmige Bewegung mit einer zweiten synchronisierten Drehung ($S_2$) erzeugen, wenn die Schüttelvorrichtung (100) in Gebrauch ist, wobei die zweite synchronisierte Drehung ($S_2$) mit der gleichen Drehgeschwindigkeit und der entgegengesetzten Drehrichtung arbeitet als die erste synchronisierte Drehung ($S_1$), wenn die Schüttelvorrichtung (100) in Gebrauch ist, wobei sich die durch die Bewegung der exzentrischen Strukturen (110, 120, 210, 220) erzeugten Drehmomente entgegensetzen und ausgleichen.

8. Schüttelvorrichtung nach Anspruch 7, wobei:

   - zwei versetzte Achsen der ersten rotierenden Baugruppe auf einer ersten Linie angeordnet sind, die zwischen ihnen die Bezugslinie (104) schneidet, und
   - zwei versetzte Achsen der zweiten rotierenden Baugruppe auf einer zweiten Linie angeordnet sind, die zwischen ihnen die Bezugslinie (104) schneidet, wobei
   - die erste Linie orthogonal zur zweiten Linie ist.

9. Schüttelvorrichtung (100) nach einem der Ansprüche 7-8, wobei die Winkelpositionen der Massenschwerpunkte der exzentrischen Strukturen (110, 120) so beschaffen sind, dass sie sich während des Gebrauchs jeweils gleichzeitig an einem Punkt befinden, der einer Bezugslinie am nächsten liegt.

10. Inkubator mit einem Schüttler (100) nach einem der Ansprüche 1-9.

11. Inkubator nach Anspruch 10, wobei die Racks jeweils eine Vielzahl von Fächern aufweisen, und wobei der Inkubator ferner Folgendes aufweist:

   - eine Vielzahl von Türen, die zu der Vielzahl von Fächern passen, und
   - einen Rotationsmechanismus, der dazu ausgelegt ist, den Schüttler (100) innerhalb des Inkubators zu drehen.

12. Inkubator nach einem der Ansprüche 10-11 mit einem Prozessor und Speichereinrichtungen, wobei ein Benutzer durch Folgendes mit dem Inkubator interagieren kann:

   - Lieferung von Identifikationsdaten an den Inkubator, um ein Rack für die Lagerung einer Substanz auf dem Rackboden abzurufen, wobei der Prozessor den benutzten Rackboden mit den Identifikationsdaten verknüpft und diese Verknüpfung in der Speichereinrichtung speichert,
   - Bereitstellen der Identifikationsdaten an den Prozessor, wobei der Prozessor die gespeicherte Position der verbundenen Substanz aus der Speichereinrichtung abruft und den Drehmechanismus betätigt, um das Rackfach mit einer passenden Tür auszurichten, und anzeigt, welche Tür zu öffnen ist oder die entsprechende Tür für den Benutzer öffnet.

13. Verfahren, das Folgendes umfasst:

   - Bereitstellen einer Schüttelvorrichtung (100) zum Rühren, Mischen oder Vermengen von Substanzen, gemäß einem der Ansprüche 1-9,
   - Aktivieren des Schüttlers durch Bewegen der Kurbeleinrichtung, wodurch das Rack in eine kreisförmige Bewegung versetzt wird, wobei sich der Massenschwerpunkt der exzentrischen Struktur (110) in dieser kreisförmigen Bewegung um eine versetzte Achse bewegt,
   - wobei das Aktivieren der Schüttelvorrichtung (100) einen Satz solcher exzentrischen Strukturen (110, 120) bewegt und so eine erste rotierende Anordnung (180) bildet, deren Kurbeleinrichtung (111, 121) eine kreisförmige Bewegung mit einer ersten synchronisierten Drehung ($S_1$) bereitstellt, wobei die rotierende Anordnung einen Massenschwerpunkt hat, der zumindest im Wesentlichen auf einer Bezugslinie (104) senkrecht zur Ebene der kreisförmigen Bewegung liegt, und wobei relative Winkelpositionen der Massenschwerpunkte der exzentrischen Strukturen (110, 120) für die rotierende Baugruppe (180) vorbestimmt sind, so dass die durch die Bewegung der exzentrischen Strukturen erzeugten Kräfte sich entgegengesetzt verhalten und entgegenwirken.

14. Verfahren nach Anspruch 13, umfassend den Schritt des Einbringens von Substanzen in die Racks von mindestens zwei der exzentrischen Strukturen.


**Revendications**

1. Agitateur (100) destiné à agiter, mélanger ou mêler intimement des substances, l'agitateur (100) comportant un corps d'agitateur (101) et l'agitateur (100) comprenant en outre :

- une structure excentrique (110) comportant :

- un moyen formant manivelle (111) propre à produire un mouvement circulaire,
- un support (113) destiné à supporter des plateaux ou des récipients et porté par le corps d'agitateur (101) par le biais d'un guide (114) empêchant une rotation du support dans le plan du mouvement circulaire du moyen formant manivelle (111), le support étant en liaison cinématique avec ledit moyen formant manivelle (111) pour recevoir ledit mouvement circulaire, un centre de masse de la structure excentrique (110) se déplaçant avec ledit mouvement circulaire autour d'un axe transposé, lorsque le moyen formant manivelle imprime le mouvement circulaire au support,

- ledit agitateur (100) comprenant un jeu de telles structures excentriques (110, 120) formant un premier ensemble rotatif (180) dont les moyens formant manivelles (111, 121) produisent un mouvement circulaire présentant une première giration synchronisée ($S_1$), durant l'utilisation de l'agitateur (100), ledit ensemble rotatif (180) ayant un centre de masse situé au moins sensiblement sur une ligne de référence (104) normale au plan du mouvement circulaire, et **caractérisé en ce que** les centres de masse des structures excentriques (110, 120) ont un emplacement angulaire le long de leurs trajectoires circulaires respectives, lesdits emplacements angulaires étant agencés de manière équiangulaire autour d'un axe transposé commun, **en ce que** les axes transposés sont réagencés de façon à être colinéaires,
les emplacements angulaires relatifs des centres de masse des structures excentriques (110, 120) le long de la trajectoire de leur mouvement circulaire étant prédéterminés pour l'ensemble rotatif (180) de telle sorte que, durant l'utilisation de l'agitateur (100), des forces produites par le mouvement des structures excentriques s'opposent et se compensent.

2. Agitateur (100) selon la revendication 1, dans lequel le moyen formant manivelle (111) comporte un maneton (112) se déplaçant en révolution autour d'un axe de giration de manivelle, ledit maneton (112) raccordant le moyen formant manivelle au support pour entraîner ledit mouvement circulaire.

3. Agitateur (100) selon l'une quelconque des revendications 1 et 2, dans lequel un cercle ayant la ligne de référence (104) en son centre a une périphérie (102) coupant chaque axe transposé.

4. Agitateur (100) selon l'une quelconque des revendications 1 à 3, dans lequel ledit moyen formant manivelle (111) est une manivelle (111) avec un maneton (112) et un axe de giration parallèle à la ligne de référence (104), la manivelle transformant sa vitesse de giration en vitesse de révolution du maneton (112).

5. Agitateur (100) selon l'une quelconque des revendications 1 à 4, dans lequel la giration synchronisée est produite par une chaîne électrocinématique commune (190) raccordée à chaque manivelle, la chaîne électrocinématique établissant une liaison cinématique entre les manivelles et des moyens d'entraînement coordonnés.

6. Agitateur (100) selon l'une quelconque des revendications 1 à 5, dans lequel la chaîne électrocinématique (190) comprend l'un au moins d'une courroie sans fin (150), d'une chaîne et d'un dispositif de commande électrique (160).

7. Agitateur (100) selon l'une quelconque des revendications 1 à 6, comprenant un second ensemble rotatif (280) comportant un second jeu de structures excentriques (210, 220), dont les moyens formant manivelles produisent un mouvement circulaire avec une seconde giration synchronisée ($S_2$), durant l'utilisation de l'agitateur (100), ladite

seconde giration synchronisée (S$_2$) se faisant à la même vitesse de giration et dans un sens de giration opposé relativement à la première giration synchronisée (S$_1$), durant l'utilisation de l'agitateur (100), de sorte que des couples produits par le mouvement des structures excentriques (110, 120, 210, 220) s'opposent et se compensent.

8. Agitateur selon la revendication 7, dans lequel :

   - deux axes transposés du premier ensemble rotatif sont disposés sur une première ligne coupant entre eux la ligne de référence (104), et
   - deux axes transposés du second ensemble rotatif sont disposés sur une seconde ligne coupant entre eux la ligne de référence (104),
   - la première ligne étant orthogonale à la seconde ligne.

9. Agitateur (100) selon l'une quelconque des revendications 7 et 8, dans lequel les emplacements angulaires des centres de masse des structures excentriques (110, 120) sont tels que, durant l'utilisation, ils se situent chacun simultanément au niveau d'un point le plus proche d'une ligne de référence.

10. Étuve comprenant un agitateur (100) selon l'une quelconque des revendications 1 à 9.

11. Étuve selon la revendication 10, dans laquelle les supports comportent chacun une pluralité d'étagères, et l'étuve comportant en outre :

    - une pluralité de portes correspondant à ladite pluralité d'étagères, et
    - un mécanisme de rotation propre à imprimer une rotation à l'agitateur (100) à l'intérieur de l'étuve.

12. Étuve selon l'une quelconque des revendications 10 et 11, comportant, en outre, un processeur et des moyens de stockage, un utilisateur pouvant interagir avec l'étuve en :

    - fournissant des données d'identification à l'étuve afin d'accéder à un support pour l'entreposage d'une substance sur l'étagère du support, le processeur associant l'étagère de support employée avec lesdites données d'identification et stockant ladite association dans lesdits moyens de stockage,
    - fournissant lesdites données d'identification au processeur, le processeur extrayant alors des moyens de stockage l'emplacement stocké associé de la substance et actionnant ledit mécanisme de rotation afin d'aligner ladite étagère de support avec une porte correspondante et indiquant à l'utilisateur la porte à ouvrir ou ouvrant la porte concernée.

13. Procédé comprenant :

    - mettre en place un agitateur (100) destiné à agiter, mélanger ou mêler intimement des substances, selon l'une quelconque des revendications 1 à 9,
    - activer l'agitateur en imprimant un mouvement au moyen formant manivelle, de sorte qu'un mouvement circulaire soit imprimé au support, le centre de masse de la structure excentrique (110) se déplaçant ainsi avec ledit mouvement circulaire autour d'un axe transposé,
    - l'activation dudit agitateur (100) imprimant un mouvement à un jeu de telles structures excentriques (110, 120) formant ainsi un premier ensemble rotatif (180), dont les moyens formant manivelles (111, 121 produisent un mouvement circulaire présentant une première giration synchronisée (S$_1$), ledit ensemble rotatif ayant un centre de masse situé au moins sensiblement sur une ligne de référence (104) normale au plan du mouvement circulaire, et des emplacements angulaires relatifs des centres de masse des structures excentriques (110, 120) étant prédéterminés pour l'ensemble rotatif (180) de telle sorte que des forces produites par le mouvement des structures excentriques s'opposent et se compensent.

14. Procédé selon la revendication 13, comprenant l'étape consistant à insérer des substances dans les supports d'au moins deux desdites structures excentriques.

Fig. 1A

Fig. 1B

Fig. 2A

Fig. 2B

Fig. 2C

Fig. 3A

Fig. 3B

Fig. 3C

Fig. 4

Fig. 5

100

163

104

111

121

120

110

115

125

161

161

162

190

160

Fig. 6

Fig. 7

Fig. 8

Fig. 9

Fig. 10

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• WO 2019222445 A1